Europäisches Patentamt

European Patent Office

Office européen des brevets

(19)

(11) Publication number: **0 137 387 B1**

(12) **EUROPEAN PATENT SPECIFICATION**

(45) Date of publication of patent specification: 04.09.91    (51) Int. Cl.⁵: **C12N 15/52**, C12N 15/77, C12P 13/22, C12N 1/20, //(C12N1/20,C12R1:13,1:15)

(21) Application number: 84111301.2

(22) Date of filing: 21.09.84

(54) Process for production of l-phenylalanine by fermentation.

(30) Priority: 22.09.83 JP 175710/83

(43) Date of publication of application:
17.04.85 Bulletin 85/16

(45) Publication of the grant of the patent:
04.09.91 Bulletin 91/36

(84) Designated Contracting States:
**DE FR GB IT**

(56) References cited:
EP-A- 0 071 023    EP-A- 0 085 958
EP-A- 0 088 166    WO-A-84/03301
FR-A- 2 103 770    FR-A- 2 482 622
GB-A- 2 053 906    GB-A- 2 076 853

**CHEMICAL ABSTRACTS, vol. 95, no. 19, 9th
November 1981, page 563, abstract no.
167135w, Columbus, Ohio, US**

(73) Proprietor: **AJINOMOTO CO., INC.
5-8, Kyobashi 1-chome, Chuo-ku
Tokyo 104(JP)**

(72) Inventor: **Nakamori, Shigeru
No. 2153-187, Kamariya-cho Kanazawa-ku
Yokohama-shi Kanagawa-ken(JP)**
Inventor: **Matsui, Kazuhiko
No. 2-20-8, Kannon Kawasaki-ku
Kawasaki-shi Kanagawa-ken(JP)**
Inventor: **Miwa, Kiyoshi
No. 3-304, Sagamidaijutaku Iwase 531
Matsudo-shi Chiba-ken(JP)**
Inventor: **Sano, Konosuke
No. 1-35-9, Uehara Shibuya-ku
Tokyo(JP)**

(74) Representative: **Strehl, Peter, Dipl.-Ing. et al
Patentanwälte Strehl Schübel-Hopf Groening
Maximilianstrasse 54 Postfach 22 14 55
W-8000 München 22(DE)**

**Description**

BACKGROUND OF THE INVENTION

Field of the Invention:

The present invention relates to a plasmid, microorganisms containing this plasmid and a process for producing L-phenylalanine by fermentation.

Description of the Prior Art:

Various prior art methods for producing L-phenylalanine by fermentation are known. For example, a method has been reported which comprises artificially inducing mutation from a wild strain to thereby impart L-phenylalanine productivity to the strain since the wild strain does not produce L-phenylalanine. Examples of mutants having L-phenylalanine productivity that have been hitherto known include mutants of the genus Brevibacterium and the genus Corynebacterium resistant to phenylalanine antagonists (U.S. Patent 3,660,235), mutants of the genus Corynebacterium requiring tyrosine for growth and resistant to phenylalanine antagonists (U.S. Patent 3,759,790), mutants of the genus Brevibacterium and the genus Corynebacterium resistant to phenylalanine antagonists and sensitive to decoinine (Japanese Published Unexamined Application 64793/81).

On the other hand, there has been recently reported an attempt to utilize genetic recombination techniques for growth of L-phenylalanine-producing bacteria, which is different from growth using articifial mutation as described above. Japanese Published Unexamined Application 1890/81, GB-A-2053906 and EP-A-0085958 disclose that mutants from Escherichia coli and genus Bacillus containing a plasmid having inserted therein a chromosomal DNA fragment obtained from the genus Escherichia and genus Bacillus, respectively, participating in the synthesis of phenylalanine produce L-phenylalanine.

Furthermore, it is known to produce amino acids by transforming a Coryneform glutamic-acid producing bacterium with a plasmid containing a gene coding for the biosynthesis of said amino acid (EP-A-0071023, GB-A-2076853). In EP-A-0136359 a document according to Article 5463), such a process is described for the production of L-phenylalanine.

SUMMARY OF THE INVENTION

It is an object of the present invention to provide a plasmid conferring increased L-phenylalanine productivity and microorganisms capable of producing L-phenylalanine with significantly increased productivity.

This and other objects of the invention as will hereinafter become more readily apparent have been accomplished by providing a plasmid conferring increased L-phenylalanine productivity obtainable from FERM-BP 580 and microorganisms having increased L-phenylalanine productivity obtainable by transforming a recipient bacterium selected from Coryneform glutamic-acid producing bacteria and mutants thereof with this plasmid. Further there is provided a process for producing L-phenylalanine, which comprises (1) culturing in a culture medium said microorganism having L-phenylalanine productivity, and (2) recovering L-phenylalanine accumulated in said culture medium. characterized in that as a microorganism having L-phenyl-alanine productivity there is used a microorganism as described above.

BRIEF DESCRIPTION OF THE DRAWING

A more complete appreciation of the invention and many of the attendant advantages thereof will be readily obtained by reference to the following detailed description when considered in connection with accompanying drawing, wherein:

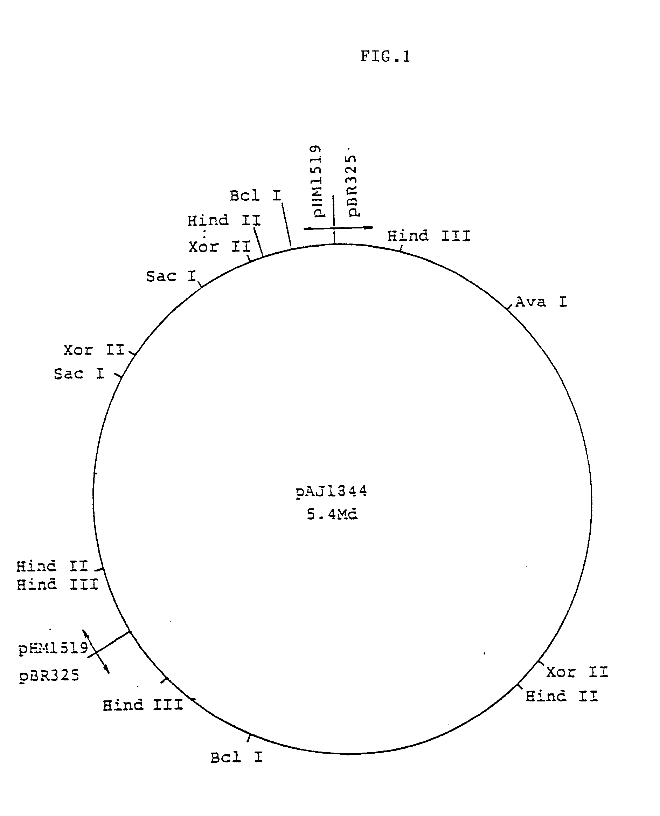

Figure 1 is a restriction map of plasmid PAJ 1844.

DESCRIPTION OF THE PREFERRED EMBODIMENTS

The present inventors have noted that by incorporating a gene connected with a vector plasmid capable of replicating in cells of a Coryneform glutamic acid-producing bacterium into a recipient bacterium selected from Coryneform glutamic acid-producing bacteria and mutants thereof, wherein the gene when introduced into a mutant induced from a Coryneform glutamic acid-producing bacterium and having auxotrophy for L-

EP 0 137 387 B1

phenylalanine is capable of eliminating the auxotrophy of the mutant for L-phenylalanine, strains capable of producing L-phenylalanine in a high yield can be obtained. These strains produce large amounts of L-phenylalanine in a conventional process for producing L-phenylalanine by fermentation as described below.

The DNA fragment containing a gene of a phenyl-alanine biosynthesis system can be obtained from chromosomal DNA of Coryneform glutamic acid-producing bacteria (bacteria that give a DNA fragment containing a gene of a phenylalanine biosynthesis system are referred to as DNA donors).

Coryneform bacteria are aerobic, Gram positive rods, are non-acid fast, and are described in Bergey's Manual of Determinative Bacteriology, 8th Edition, 599 (1974). Examples of wild strains of Coryneform glutamic acid-producing bacteria which can be utilized as host bacteria in the present invention include the following bacteria:

Brevibacterium divaricatum ATCC 14020
Brevibacterium saccarolyticum ATCC 14066
Brevibacterium immariophilum ATCC 14068
Brevibacterium lactofermentum ATCC 13869
Brevibacterium roseum ATCC 13825
Brevibacterium flavum ATCC 13826
Brevibacterium thiogenitalis ATCC 19240,
Corynebacterium acetoacidophilum ATCC 13870
Corynebacterium acetoglutamicum ATCC 15806
Corynebacterium callunae ATCC 15991
Corynebacterium glutamicum ATCC 13032 and 13060
Corynebacterium lilium ATCC 15990
Corynebacterium melassecola ATCC 17965
Corynebacterium ammoniaphilum ATCC 15354

Other host bacteria include mutants of Coryneform glutamic acid-producing bacteria which have lost glutamic acid productivity and mutants which produce products other than glutamic acid.

It is preferred to use as DNA donors mutants having enhanced biosynthesis of phenylalanine or its precursors by imparting mutation such as resistance to phenylalanine antagonists thereto, e.g. m-fluorophenylalanine. $\beta$—3-thienylalanine, and 5-methyltryptophane. The term "phenylalanine precursor" as used herein refers to at least chorismic acid and optionally other such as 3-dehydroxy-D-arabino-heptulonic acid-7-phosphate, 3-dehydroquinic acid, 3-dehydroshikimic acid, shikimic acid, shikimic acid-3-phosphate, enolpyruvylshikimic acid-3-phosphate, prephenic acid, and prephenylpyruvic acid, as well as other molecules which are interemediates in the biosynthesis of phenylalanine.

Examples of mutants having increased phenylalanine production are described in, for example, U.S. Patents 3,660,235 (Brevibacterium lactofermentum ATCC 21420 and Corynebacterium acetoacidophilum ATCC 21421) and 3,759,970 and Japanese Published Unexamined Patent Application 64793/8 (Brevibacterium lactofermentum FERM-P 5248 and 5246, Brevibacterium flavum FERM-P 5249 and Corynebacterium acetoacidophilum FERM-P 5250).

Examples of genes of the phenylalanine biosynthesis system include at least the gene for chorismic acid synthase and optionally the gene for 3-deoxy-D-arabino-heptulonic acid-7-phosphate (DAHP) synthetase, the gene for 3-dehydroquinic acid synthetase, the gene for 3-dehydroquinic acid dehydratase, the gene for shikimic acid dehydrogenase, the gene for shikimic acid kinase, the gene for 5-enolpyruvyl-shikimic acid-3-phosphate synthetase, and the gene for prephenic acid dehydratase. By inserting these genes in vector plasmid pAf1844 having the capability of autonomous replication and using the plasmids to introduce the genes into DNA recipients belonging to Coryneform glutamic acid-producing bacteria, strains having improved productivity of phenylalanine can be obtained.

As bacteria belonging to Coryneform glutamic acid-producing bacteria which can be used as DNA recipients, a variety of strains as described above can be employed. When strains having auxotrophy for phenylalanine are used as recipients, transformants having introduced genes coding for a phenylalanine biosynthesis system can be easily selected by detecting disappearance of the auxotrophy. Further in case that strains sensitive to phenylalanine antagonists are employed as recipients, transformants having introduced genes coding for a phenylalanine biosynthesis system exhibiting resistance to phenylalanine antagonists can easily be selected by monitoring resistance to phenylalanine antagonists. In order to obtain transformants having higher productivity of phenylalanine, better results are obtained using as recipients mutants in which biosynthetic activity of phenylalanine or its precursors is enhanced. This may be done by imparting to the recipient a mutation such as resistance to a phenylalanine antagonist, auxotrophy for phenylalanine, etc. Furthermore, excellent results may also be obtained with mutants in which permeability of phenylalanine out of the cells is improved, mutants in which decomposition of phenylalanine is

3

decreased, and mutants or recombinants in which biosynthetic activity of metabolites supplied for the synthesis of phenylalanine as components such as glutamine or phosphoenolpyruvate is increased.

In particular, the present invention is preferably practiced when a microorganism (produced by the process as described above) produces L-phenylalanine in an amount at least 13 mg/dl higher than that produced by the bacterium which received the gene when the microorganism and the recipient bacterium are cultivated for three days under same conditions. Increases in productivity of at least 41 mg/dl and at least 200 mg/dl are more and most preferred, respectively. Microorganisms having these increased activities can be readily recognized by measuring the amount of L-phenylalanine accumulated in the culture medium.

As the vector there is used pAf1844 which can be autonomously replicate in Coryneform glutamic acid - producing bacteria and has the following characteristics:

(a) host bacteria: Escherichia coli AJ 11883 (FERMP6519 = FERM-BP137, etc.)

(b) molecular weight: 5.4 megadaltons

(c) restriction map of restriction enzyme: see Fig. 1

(d) property: composite plasmid of pHM 1519 and pBR 325 participating in chloramphenicol resistance DNA donors or chromosomal DNAs and vector DNAs can be extracted in a conventional manner.

The chromosomal DNAs and the vector DNAs are cleaved by restriction enzymes, respectively. The cleavage of the vector DNAs is achieved either by cleaving the vector DNAs with restriction enzymes for cleaving at one site or by partially reacting restriction enzymes for cleaving at a plurality of sites with the vector DNAs. With respect to chromosomal DNAs, various kinds of restriction products can be obtained by controlling reaction conditions such that cleavage with restriction endonuclease is partially effected.

As methods for ligating the thus obtained chromosomal DNA fragments with the cleaved vector DNAs, conventional methods using ligase enzymes can be employed. On the other hand, there can be also utilized a method for ligation which comprises adding deoxyadenylic acid and deoxycytidylic acid or deoxyguanylic acid and deoxycytidylic acid to the chromosomal DNA fragments and to the cleaved vector DNAs, respectively, mixing them, and then annealing.

The incorporation of the thus obtained recombinant DNA, comprising the chromosomal DNA and the vector plasmid, into recipients belonging to Coryneform glutamic acid-producing bacteria can be done by various methods including the following: treating the recipient cells with calcium chloride to increase the permeability of DNA, as is reported regarding Escherichia coli K-12 (Mandel, M. and Higa, A., J. Mol. Biol., 53, 159 (1970)), and incorporating DNA at a stage of growth (so called competent cell) when cells become capable of incorporating DNA, as is reported for Bacillus subtilis (Duncan, C.H. , Wilson, G.A. and Young, F.E., Gene, 1, 153 (1977)). The plasmids can also be incorporated into the DNA recipients by forming protoplasts or sphereoplasts of the DNA recipients which easily incorporate plasmid DNA, as is known for Bacillus subtilis, Actinomycetes and yeast (Chang, S. and Cohen, S.N., Mole. Gen. Genet., 168, III (1979); Bibb, M.J. , Ward, J.M. and Hopwood, O.A., Nature, 274, 398 (1978); Hinnen, A. , Hicks, J.B. and Fink, G.R., Proc. Natl. Acad. Sci. USA, 75, 1929 (1978)).

In the protoplast method, a sufficiently, high frequency can be obtained even by the method used for Bacillus subtilis described above. Further, there can be properly used a method described by Japanese Published Unexamined Patent Application 57-183799 which comprises incorporating DNA into protoplasts of the genus Corynebacterium or the genus Brevibacterium in the presence of polyethylene glycol or polyvinyl alcohol and divalent metal ions. Equivalent effects can also be obtained in a method of promoting the incorporation of DNA by the addition of carboxymethyl cellulose, dextran, phycoll, Pluronic F68 (Serva Company), etc., instead of polyethylene glycol or polyvinyl alcohol.

Selection of the transformants carrying the vector plasmids containing the gene for phenylalanine biosynthesis system can be easily accomplished by selecting strains that have lost the auxotrophy for phenylalanine when phenylalanine auxotrophs are used as recipients and by selecting strains that have become resistant to the antagonists when genes for a phenylalanine biosynthesis system exhibiting phenylalanine antagonist resistance are introduced as recipients. It is also possible to select strains using improved productivity of phenylalanine as a measure. When genetic markers such as drug resistance, etc., are present on the vectors, the transformants can be more easily selected.

The methods of culturing the L-phenylalanine-producing bacteria thus obtained are conventional and are similar to methods for the cultivation of conventional L-phenylalanine-producing bacteria. That is, the culture medium can be a conventional medium containing carbon sources, nitrogen sources, inorganic ions, and, when required, minor organic nutrients such as amino acids and vitamins. Examples of carbon sources include glucose, sucrose, lactose, starch hydrolysates containing those sugars, whey, molasses, and the like. Examples of nitrogen sources include gaseous ammonia, aqueous ammonia, ammonium salts and others.

Cultivation is conducted under aerobic conditions in which the pH and the temperature of the medium are adjusted to a suitable level, and cultivation is continued until the formation and accumulation of L-phenylalanine substantially cease.

Thus, markedly high amounts of L-phenylalanine are formed and accumulated in the culture medium. To recover L-phenylalanine from the culture medium, conventional methods are applicable.

The invention now being generally described, the same will be better understood by reference to certain specific examples which are included herein for purposes of illustration only and are not intended to be limiting of the invention or any embodiment thereof, unless so specified.

Example 1

Construction of pAf 11

Chromosomal DNAs (10 μg) prepared from Brevibacterium lactofermentum AJ 3437 (FERMP-1914), a phenylalanine-producing bacterium having resistance to the phenylalanine analogue m-fluorophenylalanine, were partially cleaved with restriction enzyme Pst I. At the same time, vector plasmids pAJ 1844 (5 μg) having one site cleavable with the same enzyme and exhibiting resistance to chloramphenicol were fully cleaved with the enzyme. After inactivating the enzyme by heat treatment at 65°C for 10 minutes, both were mixed. Reaction with ligase was performed at 22°C for 2 hours and cold ethanol was added thereto. The resulting precipitates were dissolved in TEN buffer, and the resulting solution was used for protoplast transformation.

As the DNA recipient, Brevibacterium lactofermentum No. 64 (ATCC 39134) sensitive to m-fluorophenylalanine and requiring methionine, threonine and lysine for growth was used. Protoplasts of this bacterium were prepared as follows. After the strain was propagated in 5 ml of CMG medium (complete medium, glucose) at 30°C overnight, the strain was inoculated again in 5 ml of CMG medium and propagated to reach a medium exponential growth phase (absorbancy of 0.4 at 562 nm). After adding 0.6 unit/ml of penicilline G thereto (final concentration), cultivation was performed for an additional 1.5 hour, at which time 1 ml of cells were harvested. After washing with SMMP medium (0.25 M sucrose, 10 mM maleic acid, 10 mM magnesium chloride and 1.75% Penassay broth (Difco)), the cells were suspended in 1.0 ml of SMMP medium containing 10 mg/ml of lysozyme and allowed to settle at 30°C for 20 hours. After confirmation that the protoplasts were formed, the cells were harvested by centrifugation at 6000 rpm for 10 minutes. After washing with 1.0 ml of SMM (containing 0.25 M sucrose, 10 mM maleic acid and 10 mM magnesium chloride, pH 6.5) containing 0.2M $MgSO_4.7H_2O$, the protoplasts were suspended in 0.5 ml of SMMP, which was used as a protoplast suspension in experiments.

The thus prepared protoplasts, 5 μl, were mixed with 10 μl of DNAs and 10 μl of SMM having a 2-fold concentration (containing 0.5 M sucrose, 20 mM maleic acid and 20 mM magnesium chloride, pH 6.5), and 1 μl of 1 M EDTA was mixed with the resulting mixture. Then 75 μl of 40% PEG (polyethylene glycol #6000) was added to the mixture followed by mixing and settling at room temperature for 2 minutes. To the mixture, 0.5 ml of SMMP was added. After washing, the mixture was suspended in 1 ml of SMMP. In order to express genes resistant to chloramphenicol, the suspension was shaken at 30°C for 2 hours and spread on regeneration medium (containing 12 g/l of trishydroxylamine, 0.5 g/l of KCl, 10 g/l of glucose, 8.1 g/l of $MgCl_2.6H_2O$, 2.2 g/l of $CaCl_2.2H_2O$, 4 g/l of powdered yeast extract, 4 g/l of peptone, l g/l of Casamino acid (Difco Company), 0.2 g/l of $K_2HPO_4$, 135 g/l of sodium succinate, 3 μg/l of chloramphenicol and 8 g of agar, pH 7.0). After cultivation at 30°C for 1 week, the formed colonies resistant to chloramphenicol were transferred to a minimum medium containing 10 mg/dl each of methionine, threonine and lysine and 1000 μg/ml of m-fluorophenylalanine (20 g/l glucose, 10 g/l $NH_4SO_4$, 3 g/l urea, 1.0 g/l $KH_2PO_4$, 0.4 g/l $MgSO_4.7H_2O$, 0.05 g/l NaCl, 10 mg/l $FeSO_4.7H_2O$, 10 mg/l $MnSO_4$, 50 μg/l d-biotin, 200 μg/l thiamine.HCl, 10 mg/l chloramphenicol and 20 g/l agar, pH 7.0). The clones were selected from colonies grown on this plate. The plasmids isolated from the thus obtained colony AJ 12081 (FERM-P7248) (FERM-BP 580) resistant to m-fluorophenylalanine and chloramphenicol were fully cleaved with restriction enzyme Pst I. As the result of agarose gel electrophoresis, the product carried plasmids (pAJ 11) inserted with Pst I fragments of 1.2 Md and 1.4 Md derived from chromosomal DNAs of phenylalanine-producing bacteria at the cleaved region of pAJ 1844 with Pst I.

Next, transformation was performed using as a recipient chorismic acid mutase β subunit-deficient Brevibacterium lactofermentum AJ 12082 (FERM-P7249) (FERM-BP 581) requiring phenylalanine and tyrosine for growth by the method described above. The resulting strains resistant to chloramphenicol all recovered the auxotrophy for phenylalanine and tyrosine. Further, in the case of using the phenylalanine auxotrophs as an index, the production of phenylalanine was noted. As the result, it became clear that the

5

genes for coding at least chorismic acid mutase $\beta$ subunit were present on the plasmids.

Example 2

Brevibacterium lactofermentum No. 64 (ATCC 39134), phenylalaine-producing Brevibacterium lactofermentum AJ 3437 (FERMP-1914) having auxotrophy for methionine and tyrosine and Corynebacterium glutamicum (ATCC 13060) were transformed using pAJ 11 according to the protoplast transformation method described in Example 1. Thereafter, selection was performed in terms of chloramphenicol resistance to obtain AJ 12084 (FERM-p7251) (FERM-BP 583) from AJ 3437 and AJ 12083 (FERM-P7250) (FERM-BP 582) from ATCC 13060. After it was confirmed by agarose gel electrophoresis that each of the transformants carried pAJ 11, the transformants were inoculated on 20 ml of phenylalanine-producing medium (containing 130 g/l of glucose, 20 g/d of $(NH_4)_2SO_4$, 1/gl of $KH_2PO_4$, 1 g/l of $MgSO_4.7H_2O$, 10 mg/dl of $MnSO_4.7H_2O$, 50 μg/ml of biotin, 12 mg/l of thiamine.HCl, 50 ml/l of soybean protein acid hydrolysate ("Mieki"), 3 ml/l of acetic acid, 12 g/l of fumaric acid and 50 g/l of calcium carbonate; provided that 15 mg/dl each of lysine, threonine and methionine was added upon cultivation of ATCC 39134 and AJ 12081 and 40 mg/dl each of methionine and tyrosine was added upon cultivation of AJ 3437 (FERMP-1914) and AJ 12084; pH 7.0). Shake culture was performed at 30°C for 3 days and 4 days. The results are shown in Table 1.

## Table 1

| Strain | Amount of L-Phenylalanine Accumulated (mg/dl) |
|---|---|
| ATCC 39134 | 0.0 |
| AJ 12081 | 41 |
| ATCC 13060 | 2.0 |
| AJ 12083 | 15 |
| AJ 3437 | 1700 |
| AJ 12084 | 1900 |

Publications and patents cited herein are indicative of the level of skill of those skilled in the art and are herein incorporated by reference.

**Claims**

1. Plasmid pAJ11, conferring L-phenylalanine productivity, obtainable from FERM-BP 580.

2. A microorganism having L-phenylalanine productivity, obtainable by transforming a recipient bacterium selected from Coryneform glutamic acid producing bacteria and mutants thereof, with the plasmid according to claim 1.

3. A microorganism according to claim 2, wherein said recipient bacterium is a Coryneform glutamic acid producing bacterium resistant to a phenylalanine antagonist.

4. A process for producing L-phenylalanine which comprises
   (1) cultivating in a culture medium a microorganism having L-phenylalanine productivity, and
   (2) recovering the L-phenylalanine accumulated in said culture medium,
   **characterized** in that as a microorganism having L-phenylalanine productivity there is used a microorganism according to claim 2 or 3.

6

EP 0 137 387 B1

**Revendications**

1. Plasmide pAJ11 conférant la capacité de produire la L-phénylalanine, pouvant être obtenu à partir de FERM-BP 580.

2. Microorganisme ayant la capacité de produire la L-phénylalanine pouvant être obtenu par transformation d'une bactérie receveuse, choisie parmi les bactéries coryneéformes productrices d'acide glutamique et leurs mutants, avec le plasmide selon la revendication 1.

3. Microorganisme selon la revendication 2, dans lequel ladite bactérie receveuse est une bactérie coryneéforme productrice d'acide glutamique résistante à un antagoniste de la phénylalanine.

4. Procédé pour produire la L-phénylalanine qui comprend
   (1) la culture dans un milieu de culture d'un microorganisme ayant la capacité de produire la L-phénylalanine, et
   (2) la récupération de la L-phénylalanine accumulée dans ledit milieu de culture,
   caractérisé en ce que, comme microorganisme ayant la capacité de produire la L-phénylalanine, on utilise un microorganisme selon la revendication 2 ou 3.

**Patentansprüche**

1. Plasmid pAJ11, das die Fähigkeit zur Herstellung von L-Phenylalanin vermittelt und von FERM-BP 580 erhältlich ist.

2. Mikroorganismus, der die Fähigkeit zur Herstellung von L-Phenylalanin besitzt und erhältlich ist durch Transformation eines aufnehmenden Bakteriums, das unter Glutaminsäure produzierenden coryneformen Bakterien und ihren Mutanten ausgewählt ist, mit dem Plasmid nach Anspruch 1.

3. Mikroorganismus nach Anspruch 2, wobei besagtes aufnehmendes Bakterium ein Glutaminsäure produzierendes coryneformes Bakterium ist, das resistent gegen einen Phenylalanin-Antagonisten ist.

4. Verfahren zur Herstellung von L-Phenylalanin, umfassend
   (1) das Kultivieren eines Mikroorganismus mit der Fähigkeit zur Herstellung von L-Phenylalanin in einem Kulturmedium, und
   (2) das Gewinnen des in diesem Kulturmedium angereicherten L-Phenylalanins,
   dadurch gekennzeichnet, daß als Mikroorganismus mit der Fähigkeit zur Herstellung von L-Phenylalanin ein Mikroorganismus nach Anspruch 2 oder 3 verwendet wird.

7

FIG.1